(19)

Europäisches
Patentamt
European
Patent Office
Office européen
des brevets

(11) **EP 4 385 402 A1**

(12) **EUROPEAN PATENT APPLICATION**

(43) Date of publication:
**19.06.2024 Bulletin 2024/25**

(21) Application number: **23154473.5**

(22) Date of filing: **01.02.2023**

(51) International Patent Classification (IPC):
**A61B 5/024** (2006.01)  **A61B 5/318** (2021.01)
**A61B 5/369** (2021.01)  **A61B 5/389** (2021.01)
**A61B 5/398** (2021.01)  **A61B 5/00** (2006.01)

(52) Cooperative Patent Classification (CPC):
**A61B 5/02416; A61B 5/318; A61B 5/369;**
**A61B 5/389; A61B 5/398; A61B 5/4812;**
**A61B 5/4818**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **13.12.2022 US 202263432114 P**

(71) Applicant: **Koninklijke Philips N.V.**
**5656 AG Eindhoven (NL)**

(72) Inventors:
• **TIEMANN, Christian Andreas**
**5656AG Eindhoven (NL)**
• **FERREIRA DOS SANTOS DA FONSECA, Pedro Miguel**
**5656AG Eindhoven (NL)**
• **VAN DER HEIDE, Esther Marjan**
**5656AG Eindhoven (NL)**

(74) Representative: **Philips Intellectual Property & Standards**
**High Tech Campus 52**
**5656 AG Eindhoven (NL)**

(54) **HYPNODENSITY-BASED SLEEP APNEA MONITORING SYSTEM AND METHOD OF OPERATION THEREOF**

(57) A sleep monitoring system (100) including: at least one sensor (114) to sense physiological changes of a subject during a test period, the test period having a plurality of epochs, and being configured to form corresponding sensor information for the plurality of the epochs; at least one controller (120, 190, 194) to: receive the sensor information for the plurality of the epochs from each sensor; derive a hypnodensity profile for each epoch of the plurality of epochs based upon the received sensor information for the epochs; calculate at least one hypnodensity feature based upon the hypnodensity profile for each epoch of the plurality of epochs; determine a composite feature based on the at least one hypnodensity feature over the plurality of epochs; compare the composite feature to a at least one predetermined indicator related to sleep apnea; and determine a presence and/or severity of apnea based upon the comparison.

FIG. 3

**EP 4 385 402 A1**

**Description**

FIELD OF THE INVENTION

The present system relates to a system that detects the presence of sleep apnea from hypnodensity-based features derived from sleep data and, more particularly, to a portable system that assesses the presence and severity level of sleep apnea from hypnodensity-based features derived from sleep data, and a method of operation thereof.

BACKGROUND OF THE INVENTION

Sleep apnea is a disorder that disrupts breathing during sleep. People with sleep apnea temporarily stop (apnea) or decrease (hypopnea) their breathing repeatedly during sleep. The affected breathing is the result of repetitive obstruction of the airway caused by narrowing of the respiratory passages. These breathing disruptions can awaken a person or prevent deep and restful sleep and result in sleep (cycle) fragmentation. Consequently, the induced sleep fragmentation affects, among other things, daytime fatigue and sleepiness level of an affected individual. Accordingly, diagnosis of sleep apnea is important but also challenging since many people are unaware they have the condition.

Detection and diagnosis of sleep apnea is challenging for various reasons. As mentioned above, people are often unaware they have the condition because they are asleep when the primary symptoms occur such as pauses in breathing, gasping, and/or loud snoring. There are various other signs and risk factors which are suggestive for sleep apnea like daytime sleepiness, obesity, large neck circumference, age, and/or gender. Screening questionnaires can be used to assess for sleep apnea. However, the diagnostic accuracy of these symptoms and risk factors is limited. Overnight polysomnography (PSG) performed in a sleep laboratory in the presence of an attendant is currently considered to be the diagnostic standard. However, this is a costly, laborious, and cumbersome procedure which is not very suitable for screening on a large scale. PSG consists of multiple sensors to measure brain activity, as well as heart rhythm, breathing patterns, and/or arm and leg movements. Also, there must already be an initial suspicion of sleep apnea and a referral before a sleep laboratory test is conducted. Accordingly, it is difficult, if not impossible, to easily diagnose and treat sleep apnea. Home sleep monitoring could be useful to screen for sleep apnea and overcome some of abovementioned challenges, however, prior systems that could be used for home monitoring are too complicated and cumbersome to be readily applied by a typical person. Furthermore, the application of these prior systems likely affects sleep by its use and therefore, makes the diagnosis available with prior systems unreliable.

Accordingly, embodiments of the present system provide features and advantages which overcome the noted disadvantages and obviate conventional diagnostic systems and methods. To overcome the aforementioned barriers and detriments as well as others, there is a need for a mobile sleep apnea system that can be conveniently used in the home by a person to assess the presence and severity level of sleep apnea using data acquired in a home environment as well as an in a lab environment. This can result in diagnosis and treatments for individuals who would otherwise not be able to obtain proper diagnosis and treatment when it is necessary to perform testing in a laboratory.

SUMMARY OF THE INVENTION

The system(s), device(s), method(s), arrangements(s), interface(s), computer program(s), processes, etc., (hereinafter each of which will be referred to as system, unless the context indicates otherwise), described herein address problems in prior art systems.

In accordance with embodiments of the present system, there is disclosed a sleep monitoring system, including: at least one sensor configured to sense physiological changes of a subject during a test period, the test period having a plurality of epochs, and being configured to form corresponding sensor information for the plurality of the epochs; at least one controller configured to: receive the sensor information for the plurality of the epochs during the test period from each of the at least one sensor; derive a hypnodensity profile for each epoch of the plurality of epochs based upon the received sensor information for the plurality of the epochs; calculate at least one hypnodensity feature based upon the hypnodensity profile for each epoch of the plurality of epochs; determine a composite feature based on the at least one hypnodensity feature over the plurality of epochs; compare the composite feature to a at least one predetermined indicator related to sleep apnea; and determine a presence and/or severity of apnea based upon the comparison. At least one of the at least one sensor may be configured to measure sleep. The at least one controller may be further configured to monitor the at least one sensor in accordance with a test type. The sleep monitoring system may include a mobile station (MS) having a display, wherein the at least one controller may be further configured to transmit the determined presence and/or severity of apnea to the MS and to render the determined presence and/or severity of apnea on the display.

In accordance with embodiments, the at least one controller may be configured to calculate the at least one hypnodensity feature as information entropy and may be configured to determine an average of the information entropy (DA) over the plurality of epochs as the composite feature. The at least one predetermined indicator may be at least one predetermined threshold for the presence and/or the severity of apnea. The hypnodensity profile may be derived using a model of sleep classes, wherein the model may be determined from training data from a population in which at least some of the population are determined to exhibit at least some degree of sleep apnea, and wherein the at least one predetermined threshold may be determined from the training data. at least one predetermined threshold may be a first threshold value (T1), a second threshold value (T2), and a third threshold value (T3), wherein the controller may be further configured to compare the DA to the T1, T2, T3 and T4 wherein: when it is determined that the DA is less than T1, then no apnea is determined present; when it is determined that the DA is equal or greater than T1 and less than T2, then mild apnea is determined present; when it is determined that the DA is equal or greater than T2 and less than T3, then moderate apnea is determined present; and when it is determined that the DA is equal or greater than T3, severe apnea is determined present.

Further, in accordance with embodiments of the present system, there is disclosed a sleep monitoring system, including: at least one sensor configured to sense physiological changes of a subject during a test period having a plurality of epochs and form corresponding sensor information for the plurality of the epochs; at least one controller configured to: receive the sensor information for the plurality of the epochs during the test period from each of the plurality of sensors; derive a hypnodensity profile for each epoch of the plurality of epochs based upon the received sensor information for the plurality of the epochs; calculate at least one hypnodensity feature based upon the hypnodensity profile for each epoch of the plurality of epochs; form a sleep apnea prediction model based upon the calculated at least one hypnodensity feature; determine a presence and/or severity of apnea based upon the sleep apnea prediction model; and render the results of the determined presence and/or severity of apnea on a rendering device. At least one of the at least one sensor may be configured to measure sleep. The at least one controller may be further configured to monitor the at least one sensor in accordance with a test type.

In accordance with embodiments, the sleep monitoring system may include a mobile station (MS) having a display, wherein the at least one controller may be further configured to transmit the determined presence and/or severity of apnea to the MS and to render the determined presence and/or severity of apnea on the display. The at least one controller may be further configured to determine a composite feature based on the at least one hypnodensity feature over the plurality of epochs; and form the sleep apnea prediction based upon the calculated at least one hypnodensity feature and the composite feature. The at least one controller may be further configured to determine a plurality of thresholds in accordance with the determined composite feature over the plurality of epochs. The plurality of thresholds may be a first threshold value (T1), a second threshold value (T2), and a third threshold value (T3). The controller may be further configured to compare the composite feature to the T1, T2, T3 and T4 wherein: when it is determined that the composite feature is less than T1, then no apnea is determined present; and when it is determined that the composite feature is equal or greater than T1 and less than T2, then mild apnea is determined present; when it is determined that the composite feature is equal or greater than T2 and less than T3, then moderate apnea is determined present; and when it is determined that the composite feature is equal or greater than T3, then severe apnea is determined present. The at least one controller configured may be configured to calculate the at least one hypnodensity feature as information entropy and may be configured to determine the composite feature as an average of the information entropy over the plurality of epochs.

In addition, in accordance with embodiments of the present system, there is disclosed a method of monitoring sleep, the method including acts of: sensing physiological changes of a subject during a test period, the test period having a plurality of epochs and forming corresponding sensor information for the plurality of the epochs; receiving the sensor information for the plurality of the epochs during the test period from each of the at least one sensor; deriving a hypnodensity profile for each epoch of the plurality of epochs based upon the received sensor information for the plurality of the epochs; calculating at least one hypnodensity feature based upon the hypnodensity profile for each epoch of the plurality of epochs; determining a composite feature based on the at least one hypnodensity feature over the plurality of epochs; comparing the composite feature to a at least one predetermined indicator related to sleep apnea; and determining a presence and/or severity of apnea based upon the comparison. The method of monitoring sleep may include acts of: calculating the at least one hypnodensity feature as information entropy; and determining an average of the information entropy (DA) over the plurality of epochs as the composite feature. The method of monitoring sleep may include an act of determining at least one threshold, wherein the hypnodensity profile may be derived using a model of sleep classes, wherein the model may be determined from training data from a population in which at least some of the population are determined to exhibit at least some degree of sleep apnea, and wherein the at least one threshold may be determined from the training data.

## BRIEF DESCRIPTION OF THE DRAWINGS

It should be expressly understood that the drawings are included for illustrative purposes and do not represent the scope of the present system. It should also be understood that the figures may not be drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail, and by way of example, with reference to the accompanying drawings which show features of various exemplary embodiments that may be combinable and/or severable wherein:

FIG. 1 shows an illustrative schematic block diagram of a portion of a system operating in accordance with embodiments of the present system;
FIG. 2 shows an illustrative functional flow diagram performed by a process in accordance with embodiments of the present system;
FIG. 3 shows an illustrative graph including an example of a hypnodensity profile and a corresponding information entropy derived from the hypnodensity profile for a patient with no detected sleep apnea in accordance with embodiments of the present system;
FIG. 4 shows an illustrative graph including an example of a hypnodensity profile and corresponding information entropy derived from the hypnodensity profile for a patient with mild sleep apnea in accordance with embodiments of the present system;
FIG. 5 shows an illustrative graph including an example of a hypnodensity profile and corresponding information entropy derived from the hypnodensity profile for a patient with severe sleep apnea in accordance with embodiments of the present system; and
FIG. 6 shows an illustrative graph of information entropy of the hypnodensity profiles (mean $\pm$ STD) for three patients with different AHI scores in accordance with embodiments of the present system.

## DETAILED DESCRIPTION OF EMBODIMENTS

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques, and methods are omitted so as not to obscure the description of the present system.

The term "and/or," and formatives thereof, should be understood to mean that only one or more of: the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", substantially and "approximately" denote an interval of accuracy that a person skilled in the art will understand to still ensure the technical effect of the feature in question which in some cases may also denote "within engineering tolerances." For example, the terms may indicate a deviation from the indicated numerical value(s)/measurement(s)/calculation(s) of $\pm 20$ %, $\pm 15$ %, $\pm 10$ %, $\pm 5$ %, $\pm 1$ % $\pm 0.5$ % or $\pm 0.1$ %.

The terms clinician, clinicians, or formatives thereof, may include a professional such as a doctor, a clinician, a technologist, an operator, an expert, medical specialist, technician, and/or the like, who may operate and/or review information related to the patient such as system setting information (SSI), system information, and/or data generated by embodiments of the present system and associated data for review by a clinician, an expert, a medical specialist, a doctor, an operator, a technician, and/or the like unless the context indicates otherwise. The terms patient, patients, or formatives thereof, may include patients or other individuals (e.g., persons, subjects, subjects under test, etc.) or other users who may be receiving any type of sleep apnea monitoring or assessment using systems, machines, processes, and/or methods operating in accordance with embodiments of the present system.

In accordance with embodiments of the present system, the Apnea-Hypopnea Index (AHI) is an index used to indicate the severity of sleep apnea. It is represented by the number of apnea and hypopnea events per hour of sleep. In accordance with embodiments of the present system, to be considered an apnea event, the condition may persist for ten seconds or more and may be associated with a decrease in blood oxygenation. The AHI values for adults are categorized as follows: (1) Normal: AHI < 5; (2) Mild sleep apnea: $5 \leq$ AHI < 15; (3) Moderate sleep apnea: $15 \leq$ AHI< 30; and (4) Severe sleep apnea: AHI $\geq$ 30. These terms may be referred to as AHI values in the current description.

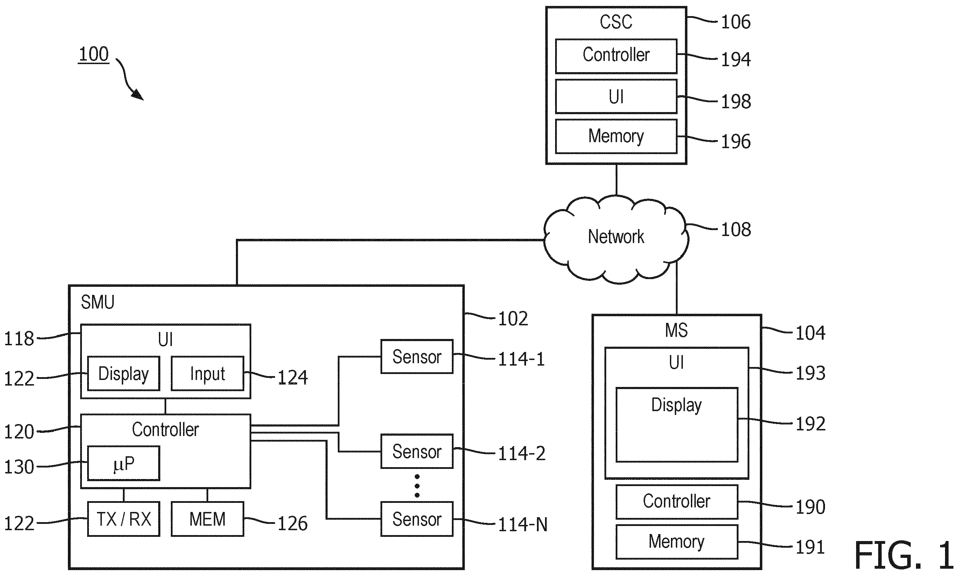

FIG. 1 shows an illustrative schematic block diagram of a portion of a system 100 (hereinafter the system 100) operating in accordance with embodiments of the present system. The system 100 may include one or more of a sleep monitoring unit (SMU) 102, a mobile station (MS) 104, and a clinical service center (CSC) 106, one or more of which may communicate with the other via any suitable communication method or methods such as wired, and/or wireless communication methods, such as over a network 108.

The network 108 may include any suitable network such as an ad-hoc network, a body area network (BAN), a personal area network (e.g., e.g., Bluetooth™, etc.), a Zigbee network, Wi-Fi, a local area network (LAN), a wide area network (WAN), a telephony network, a cellular network (e.g., 5G, etc.), etc. The SMU 102, the MS 104, the CSC 106 and the network 108 may be controlled by at least one controller (e.g., one or more of controllers 120, 190, 194 and/or one or more others) which may control the overall operation of the system and may include one or more logic devices such as a microprocessor and/or the like.

The at least one controller may access a memory and may obtain system setting information (SSI) which may include one or more of operating parameters (e.g., scan type, etc.), threshold values, warnings, display settings, user information, patient information such as patient identification (ID), content (e.g., video, audio, etc.), etc. The SSI may be set by the system and/or user and may be updated during use. The at least one controller may generate and cause to be rendered an interface with which the patient and/or clinician may, for example, interact with to, for example, set, reset, select, and/or adjust one or more settings of the SSI and/or enter information (e.g., ID, test type, settings, parameters (e.g., test duration), results, etc.) which may be stored in the SSI in a memory of the system.

The MS 104 may include any suitable mobile station(s) such as a desktop computer, laptop, smartphone (e.g., an I-Phone, a Galaxy phone, etc.), a personal digital assistant (PDA), a smart watch (e.g., an Apple™ Watch™, a Galaxy™ watch, etc.), a smart ring (e.g., generally a token) and/or the like, and may include one or more of a memory 191, at least one controller 190, a user interface (UI) 193, and a transceiver (Tx/Rx) configured to communicate with the network 108.

The UI 193 may provide an interface with which a user (e.g., the patient) may interact. Such interfaces may include, inter alia, a touch-screen display 192, an optical sensor such as a camera, a proximity sensor, a speaker, and/or a microphone. However, other user interfaces are also envisioned and intended as applicable to the present system. The UI 193 may render information, such as content, etc., audibly, visually, tactilely, etc., on a display, a speaker and/or piezoelectric device, for the convenience of the patient, and may receive information entered by the patient such as selections, requests, commands, patient settings, etc., using any suitable input method such as one or more of a gesture input, a touch input, voice input, etc. The memory 191 may store information for use by the system such as the SSI, SMI, system settings, system parameters, operating parameters, and/or combinations thereof, as discussed herein. The patient identification (ID) may be employed to identify the patient and/or the corresponding test results. The MS 104 may transmit information such as operating parameters, instructions, requests, commands (e.g., a synchronization command, etc.), to the SMU 102 and/or the CSC 106. The MS 104 may receive information generated by and/or otherwise transmitted by the SMU 102 and/or the CSC 106, such as the SSI, operating parameters, patient data, such as sleep monitoring information (SMI), and/or combinations thereof, for further processing. The MS 104 may further receive and/or transmit the SMI as discussed herein. Data transfer may occur on periodic and/or non-periodic intervals as may be set forth in the SSI and/or as requested by the system (e.g., the SMU 102, the CSC 106, and/or the MS 104), patient, and/or clinician. For the sake of clarity, only a single MS 104 is discussed unless the context indicates otherwise. However, without limitation one or more other MSs may be employed by the system. For example, a clinician such as a doctor may have an MS which may receive information such as SSI, SMI, etc. from other portions of the system for further review, processing, and/or storage. Further, a patient may have more than a single MS, one or more of which may receive information such as SSI, SMI, etc. from other portions of the system for further review, processing, and/or storage.

The CSC 106 may include one or more of a controller 194, a memory 196, and a UI 198. The controller 194 may control the overall operation of the CSC 106 and, as such may receive information, such as the SSI, SMI, and/or other information from the SMU 102 and/or the MS 104 for further processing. The CSC 106 may further communicate with the SMU 102 and/or MS 104 to, for example, transmit diagnostic results of the processing (e.g., apnea detected, apnea not detected, etc.) to be rendered on a rendering device of the MS 104 for the convenience of the user. The diagnostic results may be stored as the SMI in a memory of the system. The CSC 106 may process data in real time and/or may obtain data that has been stored in a memory of the system.

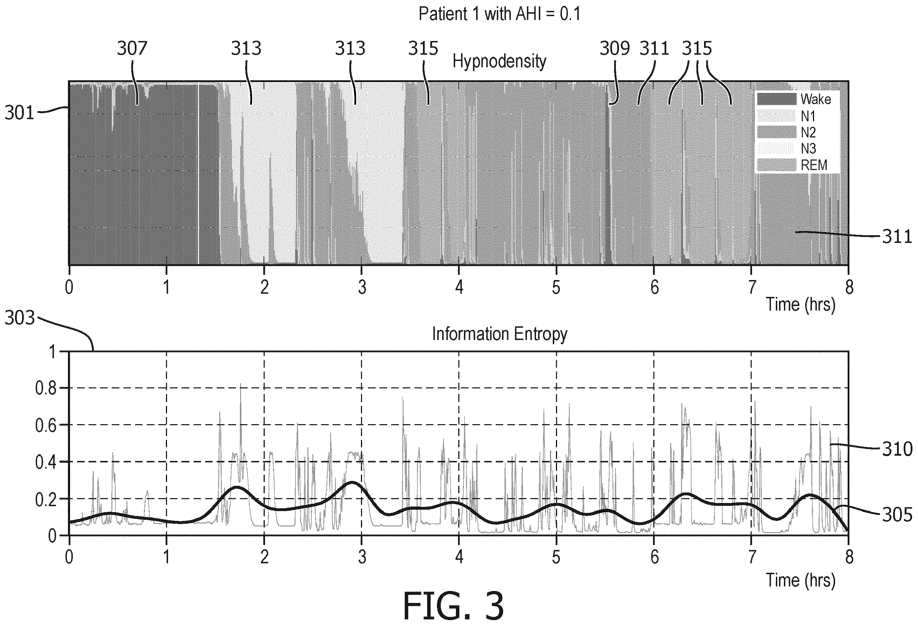

One or more controllers of the system 100 may employ machine learning and/or artificial intelligence (AI) engines to process information generated by the system, such as the SMI, as well as data provided to the system, such as historical information. Further, the one or more controllers of the system 100 may be operative to detect for example, sleep stage probabilities (e.g., see, FIGs. 3-5), whether a patient has sleep apnea, the severity of sleep apnea, etc., and/or may store and/or update results in a memory of the system (e.g., within the SMI), and may forward these results to the corresponding patient or clinician for their convenience as may be set forth in the SSI. The SMI may be accessed by the CSC 106, the MS 104, and/or the SMU 102 of the system for further review, processing, and/or analysis.

The SMU 102 may include one or more of a controller 120, a Tx/Rx 122, a memory 126, sensors 114-1 through 114-N (generally sensors 114, where N is an integer) and a user interface (UI) 118 with which a user, such as the patient or clinician, may interact. The controller 120 may control the overall operation of the SMU 102 and may communicate with one or more of the CSC 106, the MS 104, and the network 108 via the Tx/Rx 122. The controller 120 may include one or more logic devices such as a microprocessor ($\mu$P) 130 and may control the overall operation of the SMU 102. It should be appreciated that in some embodiments the controller 130 may include digital and/or analog control circuitry. It is further envisioned that the SMU 102, the MS 104 and/or the CSC 106 may be stand-alone units, for example with its own corresponding power supply.

The UI 118 may include a display 122 (e.g., a touch-screen display) and a user input interface 124 which may be combined with or separate from each other. The user input interface 124 may include a keyboard, a mouse, a trackball, touch sensitive sensor, and/or other device, such as a touch-sensitive display, which may be stand alone or may be a part of a system, such as part of a laptop, a personal digital assistant (PDA), a mobile phone (e.g., a smartphone), a smart watch, an e-reader, a monitor, a smart or dumb terminal, a smart headband, a token or other device for communicating with the controller 120 via any operable link such as an wired, and/or wireless (e.g., RF, optical, etc.) communication link. The user input interface 124 may be operable for interacting with the controller 120 including enabling interaction within the UI 118 as described herein. Clearly the controller 120, the sensors 114, the user input interface 124, the user interface (UI) 118, the memory 126 and the network 108 may all or partly be a portion of a computer system or other device.

The UI 118 may be operative to provide audio/visual feedback as well as graphical user interfaces (GUIs) to the user of the present system and may inform the operator of operating parameters, operating states, etc. The controller 120 may be operative to render information, such as still or video information, as well as audio information on a rendering device of the system such as on the display 122 and/or speaker of the UI 118. This information may include information related to operating parameters, instructions, feedback, patient information and/or other information related to an operation of the system, or portions thereof, such as SSI or portions thereof, SMI, clinical decision support, application data, data generated by the system, operating parameters, etc., and/or combinations thereof. Clinical decision support (CDS) may be employed to analyze data generated by the system and may generate determinations which may be stored in the system and/or may be rendered for the convenience of the patient and/or clinician depending upon settings in the SSI. The SSI may be system setting information that may be used by the system so set operational parameters and settings as may be set by the system and/or the user.

The controller 120 may be operable for providing control signals and/or performing operations in response to input signals from the user input device 124 as well as in response to other devices, such as the sensors 114, and executing instructions stored in the memory 126. The $\mu$P 130 may include one or more of a microprocessor, an application-specific and/or general-use integrated circuit(s), a logic device, etc. Further, the $\mu$P 130 may be a dedicated processor for performing in accordance with the present system and/or may be a general-purpose processor wherein only one of many functions operates for performing in accordance with the present system. The $\mu$P 130 may operate utilizing a program portion, multiple program segments, and/or may be a hardware device utilizing a dedicated and/or multipurpose integrated circuit. It is envisioned that one or more portions of the SMU 102, such as the controller 120, may be operationally coupled to the memory 126, the user interface (UI) 118 including a rendering device such as the display 122, the sensors 114, the user input interface 124, and the network 108.

The memory 126 may include any suitable memory configured to store information used, received and/or generated by the SMU 102, such as operating instructions, application data, as well as other data related to the described operation such as application data, data generated by the system, operating parameters, SSI, SMI, etc., and/or combinations thereof. The application data, data generated by the system, operating parameters, SSI, SMI, etc., and/or combinations thereof, may be received by the controller 120 for configuring (e.g., programming) the controller 120 to perform operation acts in accordance with the present system. The controller 120 so configured becomes a special purpose machine particularly suited for performing in accordance with embodiments of the present system. In some embodiments, the memory 126 may store information related to test types such as sensors employed by the test type, instructions for running the test type, the operating flow of the test type, SSI and/or SMI generated by the test type.

The methods of the present system are particularly suited to be carried out by a computer software program, such program containing modules corresponding to one or more of the individual steps or acts described and/or envisioned by the present system. Such program may of course be embodied in a computer-readable medium, such as an integrated chip, a peripheral device and/or memory, such as the memory 126 or another memory coupled to the controller 120. The program and/or program portions, for example contained in the memory 126, may configure the controller 120 to implement the methods, operational acts, and functions disclosed herein. The memories may be distributed, for example between the clients and/or servers, or local, and the controller 120, where additional processors may be provided, may also be distributed or may be singular. The memories may be implemented as electrical, magnetic, or optical memory and/or any combination of these or other types of storage devices. Moreover, the term "memory" should be construed broadly enough to encompass any information able to be read from and/or written to an address in an addressable space accessible by the $\mu$P 130 of the controller 120. With this definition, information accessible through a network, such as the network 108, is still within the memory, for instance, because the $\mu$P 130 may retrieve the information from the network 108 for operation in accordance with embodiments of the present system.

The SMU 102 may include any suitable measurement system or systems to monitor the patient's physiological changes over time (e.g., epochs) using one or more of the sensors 114. The sensors 114 may include electrode type sensors to sense a patient's physiology such as heart rhythm, brain activity, etc. The sensors 114 may include an optical type sensor (or sensors) to determine (peripheral) oxygen saturation (SPO2), electromyography signals (EMG), patient image information (e.g., such as when the optical sensor is incorporated into a camera, etc.). For example, one or more EMG sensors may be configured to sense electrical signals at the skin of the patient and provide these electrical signals to the controller 120 for further processing. The sensors 114 may include an accelerometer, gyroscope and/or another motion type sensor to determine movement of the patient. The sensors 114 may include a microphone to capture, for example, snoring of the patient during testing. As readily appreciated, one or more other sensors or sensor types may be employed in accordance with embodiments of the present system.

Further, although three sensors 114 are shown for illustration, there may be other numbers of these sensors in accordance with embodiments, such as one or more sensors, which may be utilized in accordance with embodiments of the present system and are typically selected based on the one or more test types that are being performed. In addition, while the sensor 114 is illustratively shown as a portion of the SMU 102, in accordance with embodiments, the sensor 114 may be a part of the MS 104. For example, in a case wherein the MS is a smart device, such as a smartphone, smartwatch, smart ring (e.g., generally, a token), the smart device may include one or more sensors 114 configured to sense physiological information which may be utilized in accordance with the present system the same or similar to when the sensor 114 is a part of the SMU 102. Nonetheless, to simplify the discussion herein, the sensor 114 is discussed as a portion of the SMU 102, however, the discussion should be understood to encompass the sensor 114 being separate from the SMU 102 (e.g., standalone, a portion of the MS 104, etc.).

For example, it is envisioned that when employing the EEG test type for example, one or more electrodes, such as may be provided by a Philips™ SmartSleep™ headband or the like, may be employed. In some embodiments, one or more functions of the patient during sleep may be tracked with other modalities either alone, or in combination. For example, EEG signal(s) may be captured, along with, heart rate variability (e.g., which may be measured with one or more of ECG, PPG, bed sensors, remote PPG cameras, etc.) and/or respiratory variability (e.g., measured with surrogate measures of respiratory effort based on bioimpedance, thoracic or abdominal belts, PPG blood volume amplitude variations, bed sensors, radio-frequency (RF)/Doppler radar, infrared cameras, etc.). Thus, the sensors 114 of the system may vary based upon the test type and/or system and/or patient preferences. For example, the sensors 114 may be selected in accordance with the (sleep) test type employed to track the one or more bodily functions of the patient during sleep. Similarly, the controller 120 may operate in accordance with the type of sleep test or tests employed to track the one or more signals corresponding to the physiological signals of the patient over the study period and may process these signals in accordance with the test type. In some embodiments, when performing a PSG-type test for example, the sensors 114 may record one or more of eye movement, oxygen and/or carbon dioxide levels in the blood, heart rate, rhythm, breathing rate and rhythm, and airflow through the mouth and nose, and snoring and form corresponding sensor information that may be processed in accordance with the PSG-type test to determine the respiratory status of the patient which may then be stored in the SMI along with the processed signals, etc.

The sensors 114 may be situated at one or more portions of the system and may sense related parameters, form corresponding sensor information, and provide this sensor information to the controller 120 for further processing. Accordingly, the sensors 114 may include sensors which may be specific to a (sleep) test type being performed. The sensors 114 may be distributed throughout the system 100.

During a study period, one or more test types may utilize corresponding one or more sensors 114 to determine physiological changes of the patient from which sleep features of the patient may be derived. These test types may have process flows and operations that may be stored in the SSI and may be selected by the SSI, the patient, and/or the clinician as may be desired. In accordance with embodiments of the present system, it is envisioned that the SMU 102 may monitor the patient's physiology over a study period using one or more test types (e.g., each corresponding to a different type of sleep study), form corresponding sensor information which may be processed in accordance with the corresponding test type and thereafter stored as test results in the SMI. The study period may be a time period during which the patient is monitored and, in the present embodiments, may be set to an eight-hour period which may be broken down into epochs, such as of 30 second intervals. While epochs may be 30 seconds, however, in accordance with embodiments of the present system, there is no restriction on the duration. Accordingly, it should be understood that other values may be set for either or both of the study period and the epochs (e.g., study period = 6 hours, and epochs = one minute, etc.) as may be desired.

These test types may each employ one of more sensor types with specific type, placement and operation that may correspond with the test type. For example, while both Electroencephalography (EEG) and electromyography (EMG) test types may employ electrode type sensors (e.g., electrode patches, electrode bands, etc.), the EEG test may utilize electrodes placed along the scalp of the patient to record bio signals representing electrical activity of the brain, the EMG test may place the electrodes along, or on, the chest of the patient to record bio signals from skeletal muscles. Further, the EMG test type may also utilize an optical sensor placed on the chest of the patient in place of the electrodes or together with the electrodes. In either event, these bio signals that are captured by the sensors 114 may then be processed in accordance with their corresponding test type (e.g., the EMG) and stored in the SMI for later use. Suitable test types (e.g., types of sleep studies) may include a home-type sleep study (e.g., using a simple test with few sensors such as used by the EMG type test that may monitor skeletal muscles, etc.), a Polysomnography (PSG) -type study which may monitor one or more bodily functions (e.g., for physiological changes) of the patient, such as brain activity (e.g., using an Electroencephalography (EEG) -type study), eye movements (e.g., using an Electrooculography (EOG) type study), muscle activity (e.g., using an electromyography (EMG) -type study), and/or heart rhythm (e.g., electrocardiography (ECG) -type study).

In accordance with embodiments of the present system, in response to one or more tests, corresponding sensor information may be formed into an electrogram, electrooculogram, electromyogram, electrocardiogram, etc., respectively, depending upon study type, and may be stored in the SMI and/or be transmitted to one or more controllers (e.g., see, controller 120, controller 190, controller 194, and/or other controller) for further processing. For example, the controller 120 may be configured to communicate with the MS 104 using any suitable communication method or methods such as via a wireless and/or wired communication method (e.g., via an RF connection, such as a Bluetooth™ connection, etc.). The controller 120 may obtain SSI from the MS 104 and store this information in the memory 126 for reference at a later time and/or to configure its operation in accordance with the SSI. During operation, the controller 120 may, for example, receive analog signals from the sensors 114, amplify (e.g., using one or more amplifiers) and/or condition these analog signals (e.g., using one or more signal conditioners), convert these signals (e.g., using one or more analog-to-digital (A/D) converters) to digital signals (e.g., at a desired sampling rate (as may be set in the SSI)) to form corresponding information which may be further processed and/or may be stored in the SMI for later use and/or processing to, for example, determine the respiratory status of the patient. In accordance with embodiments, the sensors may capture signals as digital signals and thereafter be suitably processed, stored, etc. For example, in accordance with embodiments, one or more of the sensors 114 may be an optical sensor (e.g., of a camera) that capture images of the patient as digital images that are processed, stored, etc.

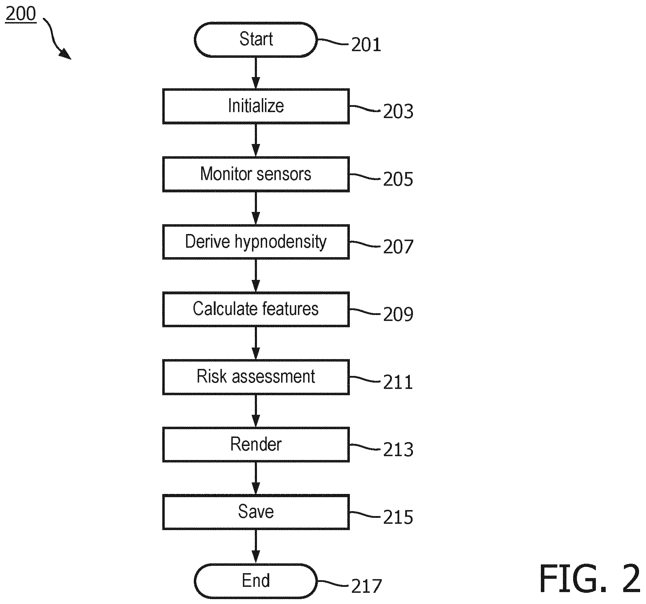

The process in accordance with embodiments may apply machine learning algorithms or the like to analyze one or more of the electrogram, electrooculogram, electromyogram, electrocardiogram, etc., and derive, or otherwise determine, a hypnodensity profile for each sleep stage per epoch (e.g., using a 30 second epoch) over the testing period (e.g., sleep session) as described herein, such as with reference to the process of FIG. 2. The hypnodensity profile in accordance with embodiments of the present system shows a probability distribution per stage of sleep for a patient/subject representing an indication of how likely a patient is in a particular stage of sleep during an epoch. In accordance with embodiments, per epoch, there is a probability value for each sleep stage class (e.g., wake, REM, etc.), which total probability for each (separate) class combined add up to one (1). Based upon this hypnodensity profile, the machine learning algorithm in accordance with embodiments may determine the information entropy contained in the received signal. The general description of information entropy is given by:

$$H(X) = -\sum_{x \in X} p(x) \log_b p(x). \qquad \text{(Equation 1)}$$

In accordance with embodiments of the present system, the information entropy may be calculated for each epoch. Here p is a vector containing the probability for each sleep stage class $x$ in the total set of sleep stage classes X. Thus, the information entropy may be derived from the hypnodensity profile. In some embodiments, training data may be employed to build one or more models which the machine learning algorithm may use for the derivation.

An example of an operational process performed by the system are described with reference to FIG. 2 which shows an illustrative functional flow diagram performed by a process 200 in accordance with embodiments of the present system. The process 200 may be performed using one or more processors, computers, controllers, etc., communicating over a network and may obtain information from, and/or store information to, one or more memories which may be local and/or remote from each other. The process 200 may include one of more of the following acts. In accordance with embodiments of the present system, the acts of process 200 may be performed using a controller operating in accordance with embodiments of the present system. Further, one or more of these acts may be combined and/or separated into sub-acts, or performed serially or in parallel, as may be desired. Further, one or more of these acts may be skipped depending upon settings. For the sake of clarity, the process may be described with reference to a single SMU and MS. However, without limitation, it should be understood that the process may employ a plurality of one or more of SMUs, MSs, etc., each of which may include a separate workflow such as a sub-workflow. In operation, the process 200 may start during act 201 and then proceed to act 203.

During act 203, the process may be initialized by performing an initialization routine. During initialization, the process may load SSI and may set system settings in accordance with the SSI. The process may further obtain machine learning model(s) and/or algorithm(s) that may process data generated by the process 200 in accordance with embodiments of the present system. The one or more models and/or algorithms may be derived using training data from people that are determined to exhibit a given physical condition, such as sleep apnea, and may indicate its severity. The SSI may further include information related to the duration of the study period (e.g., eight hours, although other periods are also envisioned), epoch intervals (e.g., 30 seconds, although other epochs such as one minute, etc., are also envisioned), test process flow, test sensor parameters, and/or other settings.

After completing act 203, the process may continue to act 205 during which the sensors (e.g., such as one or more electrodes, optical sensors, etc.) may be monitored in accordance with the test type(s) (e.g., ECG for ECG-type tests, EMR for EMR-type tests, etc.) to obtain sensor information indicative of the patient's physiological signals over time, such as during the study period (e.g., eight hours or until terminated by a clinician or patient whichever is sooner in the present embodiments) in accordance with one or more test types being performed. The sensor information may be processed (e.g., by the one or more controllers) in accordance with the corresponding test type and thereafter stored as test results in the SMI. These test types may employ one of more sensor types with specific placement and/or operation that may correspond with the test type being performed as described herein. For example, when using EMG tests, the process may obtain the sensor information from the EMG sensors such as surface EMG signals. Likewise, the process may obtain an audio signal from a microphone and store this as audio information.

When using an EEG-type test, the process may monitor the sleep of the patient using sensors with single or multiple electrodes. In some embodiments, it is envisioned that the Philips™ SmartSleep™ headband may be employed. However, it is also envisioned that more extensive testing methods of different test types may be employed for more in-depth monitoring like PSG including EEG, EOG, and EMG. Alternatively, other test types may include further sleep features that may be tracked with other modalities separately or in combination with EEG, such as heart rate variability (e.g., measured with ECG, PPG, acceleration/IMU sensors, bed sensors, remote PPG cameras, etc.), respiratory variability (e.g., measure with surrogate measures of respiratory effort based on bioimpedance, thoracic or abdominal belts, PPG blood volume amplitude variations, bed sensors, RF/Doppler radar, infrared cameras, acceleration/IMU sensors, etc.), etc. During the duration of the testing period, the process may monitor the sensors that are being employed, form corresponding sensor information, and store this information in a memory of the system such as in the SMI for further processing and analysis.

After completing act 205, the process may continue to act 207 during which the process (e.g., a controller) may derive a hypnodensity profile for each sleep stage, divided into epochs (e.g., using a 30 second epoch) over the testing period (e.g., sleep session) from the sensor information. The hypnodensity profile provides a probability for each sleep stage that may be determined for each 30-second epoch. Thus, from the hypnodensity profile, one or more probability values may be determined that indicate the probability that the patient is in a given one or more sleep stage(s) for each given epoch. The higher the probability determined for a sleep stage, the more likely the patient is in that sleep stage. Thus, there may be one or more sleep stages with a corresponding probability value per epoch. The hypnodensity profile of a sleep session (e.g., the testing duration) may be derived by integrating data from a plurality of data sources (e.g., see, FIG. 1, sensors 114), to determine the sensor information (e.g., SMI) collected over the duration of the testing period. Thereafter, from the SMI, a machine learning model may be used to analyze the SMI to determine the hypodensity profile. For example, a machine learning model, such as employed by Philips™ Somnolyzer™ software as an example using PSG or EEG data, may be utilized to determine the hypodensity profile.

As contemplated, other kinds of models using other and/or additional types of data may be suitably applied. In accordance with embodiments of the present system, such a model would take certain sensor data as input, propagate this through a given network architecture (e.g., developed based on training data), followed by an output layer containing nodes for the probabilities for the sleep stage classes. For example, a given type of sensor data and/or result of the sensor data, may be analyzed to determine a likelihood that the data for a given epoch during the testing period correlates to a given sleep stage (e.g., that the data indicates a given sleep stage). By training the model using training data with indications of what the determined sleep stage was for the training data, a model may be produced that indicates a probability value (e.g., with all the probability values for all the sleep stages adding up to 1 as discussed herein) that indicates a probability that a given one or more sleep stages is indicated by a given portion (e.g., an epoch) of the sensor data (e.g., SMI) collected over the duration of the testing period. In this way, the present system provides improvements over what heretofore was possible using previous system, by providing a system that may be scaled to the scope of the current problem that exists in the population at large regarding undiagnosed medical conditions, such as sleep apnea.

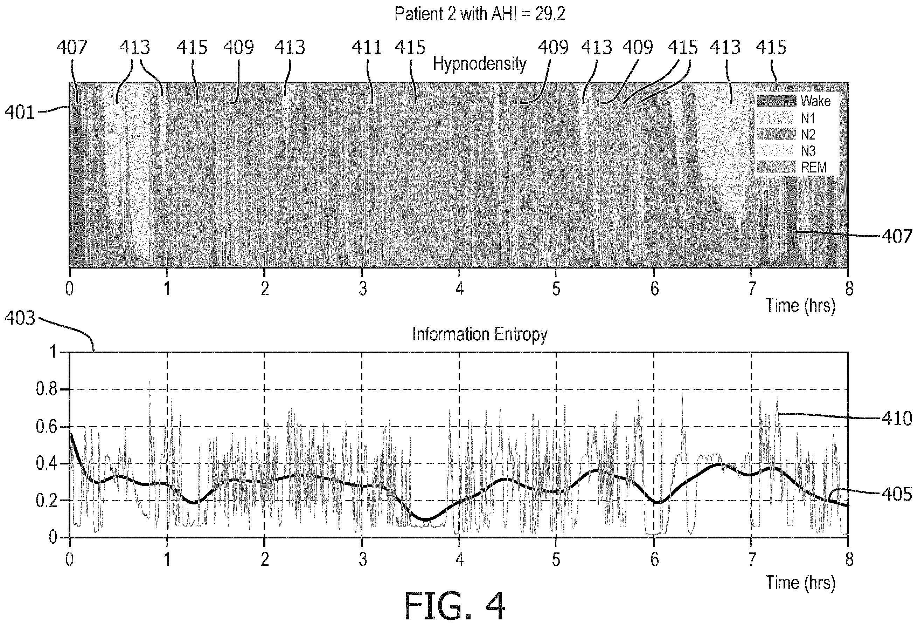

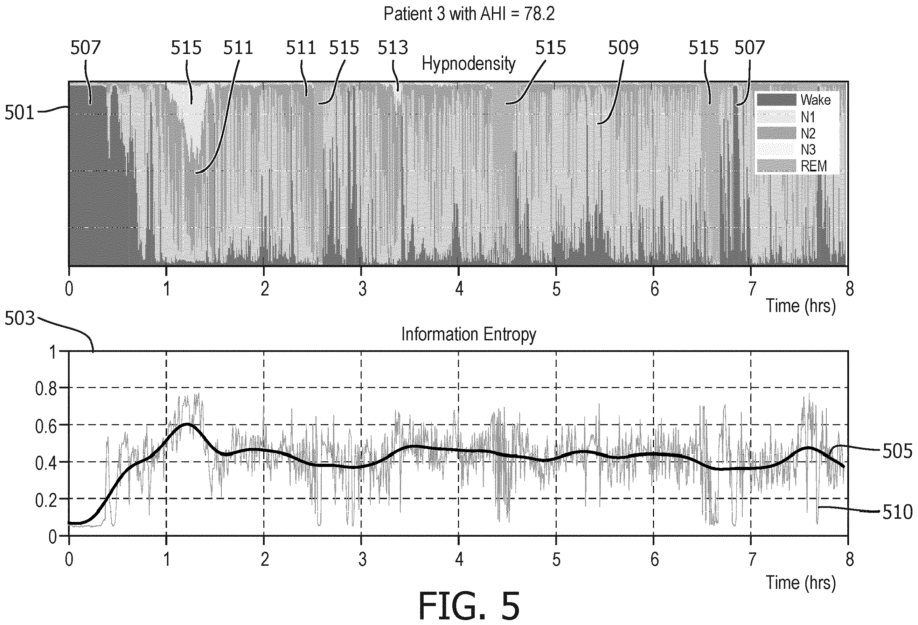

A plurality of graphs illustrating hypnodensity profiles for patients with normal sleep (e.g., no diagnosed apnea) (e.g., patient 1), moderate sleep apnea (e.g., patient 2), and severe sleep apnea (e.g., patient 3) are described with reference to FIGs. 3, 4, and 5, respectively. FIG. 3 shows an illustrative graph including an example of a hypnodensity profile (graph 301) and a corresponding information entropy (graph 303) derived from the hypnodensity profile for a patient with no detected sleep apnea in accordance with embodiments of the present system. FIG. 4 shows an illustrative graph including an example of a hypnodensity profile (graph 401) and corresponding information entropy (graph 403) derived from the hypnodensity profile for a patient with mild sleep apnea in accordance with embodiments of the present system. FIG. 5 shows an illustrative graph including an example of a hypnodensity profile (graph 501) and corresponding information entropy (graph 503) derived from the hypnodensity profile for a patient with severe sleep apnea in accordance with embodiments of the present system. In each of these graphs, a plurality of sleep stages such as stage 1, stage 2, stage 3, and stage 4, sleep cycles are illustrated as N1 (309, 409, 509), N2 (311, 411, 511), N3 (313, 413, 513,), and rapid eye movement (REM) sleep (315, 415, 515) cycles, respectively. The wake cycles are illustrated as (wake, 307, 407, 507). Further, the information entropy is illustrated in graphs 303, 403, 503, with an entropy value shown calculated per epoch thereby, producing timeseries graphs of entropy values 310, 410, 510 (e.g., the light gray lines shown in graphs 303, 403, 503 respectively in FIGs. 3, 4, 5). Information entropy graphs 305, 405, 505, are illustratively shown as smoothed averages of the calculated entropy values which helps to visualize the differences between the patients illustratively shown in FIGs. 3, 4, 5.

Illustratively, FIGs. 3, 4, 5 show hypnodensity profiles based on 5 sleep classes (Wake, N1, N2, N3, REM). In accordance with the present system, a given number of sleep classes is not a strict requirement. Depending on the measurement setup (e.g., number and/or type of sensors provided, environment of testing, etc.), it might not be possible to determine probabilities for all sleep stages. For instance, N1 and N2 may be combined into one "light sleep" class. This would result in a hypnodensity profile based on four (4) classes which is not a problem for the present system. As readily appreciated, in evaluating a condition such as sleep apnea, more or less classes (groupings) may be determined without departing from the spirit and scope of the present system.

With reference to FIG. 3, the sleep cycles are illustrated as wake (307), N1 (309), N2 (311), N3 (313), and REM sleep (315) cycles, respectively. For the patient illustratively monitored to produce FIG. 3 that has no detected sleep apnea, there is little ambiguity in the sleep stage probabilities. For many epochs during the testing period, a dominant sleep stage probability is present. Hence the sleep cycles (e.g., wake, N1, N2, N3 and REM) are well-defined (e.g., shown with a high probability) and there is practically no sleep fragmentation visible (multiple sleep stages for given epochs). As shown, there are clear and typical cycles of deep sleep (as illustrated by the N3 sleep cycles 313) early in the testing period, followed by rapid eye movement (REM) cycles (315) later in the test. In this graph, the Apnea-Hypopnea Index (AHI) for the patient may be illustratively 0.1 (normal) and the patient has no detected sleep apnea. With reference to the graph 303, the information entropy (305) is illustrated and is relatively low. The AHI is calculated by counting the number of identified apneas and hypopneas during sleep and dividing this number by the hours of sleep (sum of N1, N2, N3, REM).

With reference to FIG. 4, the sleep cycles are illustrated as wake (407), N1 (409), N2 (411), N3 (413) and REM sleep (415) cycles, respectively. For the patient illustratively monitored to produce FIG. 4 that has moderate sleep apnea, there is greater ambiguity in the sleep stage probabilities when compared to the graph 301 of FIG. 3. For many epochs during the testing period, a dominant sleep stage probability is less prevalent. Hence the sleep cycles (e.g., wake, N1, N2, N3, and REM) are not as well-defined as the example shown in graph 301 of FIG. 3 and sleep fragmentation is clearly visible. For example, the later REM sleep cycles 415 are broken in, for example, the fifth through sixth, and seventh hours of the testing period. Cycles of deep sleep are fragmented (as illustrated by the N3 sleep cycles 413) early and later in the testing period as are the REM cycles (415) which appear later in the testing period. There are also more N1 cycles (409) interspersed with N2 cycles (411) during many epochs not having a dominant sleep stage. In this graph (e.g., 401), the AHI for the patient that has moderate sleep apnea may be illustratively 29.2. With reference to the graph 403, the information entropy (405) is illustrated and is higher than the information entropy shown in graph 303.

With reference to FIG. 5, the sleep cycles are illustrated as wake (507), N1 (509), N2 (511), N3 (513), and REM sleep (515) cycles, respectively. For the patient illustratively monitored to produce FIG. 5 that has severe sleep apnea, the wake cycle (wake (507)) is clearly broken and interspersed throughout the testing period. There is greater ambiguity in the sleep stage probabilities when compared to the graphs 301 and 401 of FIGs. 3, 4, respectively. For many epochs during the testing period, a dominant sleep stage probability is much less prevalent than in the graphs 301, 401. Hence the sleep cycles (e.g., wake, N1, N2, N3 and REM) are not as well-defined as the examples shown in graphs 301, 401 of FIGs. 3, 4, respectively, and sleep fragmentation is clearly visible. For example, the later REM sleep cycles 515 are broken and interspersed in, for example, the second, fourth, and fifth hours of the testing period. The cycles of deep sleep are short and fragmented (as illustrated by the N3 sleep cycles 513). The REM cycles (515) are shorter and fragmented. There are also more N1 cycles (509) interspersed with N2 cycles (511) with many epochs not having a dominant sleep stage. In this graph (e.g., 501), the AHI for the patient that has severe sleep apnea may be illustratively 78.2. With reference to the graph 503, the information entropy (505) is illustrated and is higher than the information entropy shown in graphs 303, 403 shown in FIGs. 3, 4, respectively.

Referring back to FIG. 2, after completing act 207, the process may continue to act 209 during which the hypnodensity-based features and statistics from the hypnodensity profile may be calculated. Various features and statistics may be calculated from the hypnodensity profile (e.g., derived during act 207 for each corresponding patient) providing information on the presence and severity of sleep apnea. The unpredictability of the sleep stages as illustrated in the above examples (e.g., see act 207 and FIGs. 3, 4, 5) may be captured via an information entropy metric (e.g., see, H(X) calculation shown in Equation 1, herein), where a higher value of the information entropy metric indicates less predictability that a sleep stage has occurred. For example, in the graphs 303, 403, 503 respectively shown in FIGs. 3, 4, 5, there is shown an entropy value calculated per epoch shown as the timeseries of entropy values (graphs 310. 410. 510), with the information entropy graphs 305, 405, 505 being illustratively shown as smoothed averages of the entropy values which helps to visualize the differences between the illustrative patients. For example, the calculated entropy of sleep apnea being present for each patient shown by the information entropy graphs 305, 405, 505 (e.g., the ordinate of the graphs) is higher for the patients that have more severe sleep apnea than for patients that have comparatively less severe sleep apnea. Illustratively, the entropy calculation for a certain epoch is based on the 5 (number of sleep stage classes) probability values as illustratively shown for calculating H(X) in Equation 1, herein. This calculation may be modified to normalize the entropy between 0 and 1, which makes the graph a bit easier to interpret. For example, the entropy may be normalized between 0 and 1. In this example, all sleep stages have equal probability (all 5 stages at 20%) and the entropy is 1. Following this example, in the case 1 sleep stage is at 100%, the entropy is 0.

Hence, the information entropy is one example of a feature that may be used to assess the presence of sleep apnea. The graphs (e.g., see, 303, 403, and 503 of FIGs. 3, 4, 5, respectively) illustrate the information entropy of the respective corresponding hypnodensity profiles (e.g., see 301, 401, 501, respectively). With reference to the graphs 303, 403, 503, it is seen that as the sleep stage becomes less predictable, the information entropy metric increases (c.f., graphs 305, 405, and 505). For the patient without sleep apnea (e.g., patient 1), the information entropy metric is shown as consistently low (e.g., see, graph 305), especially as illustrated in the smoothed line (graph 305). In the raw entropy calculated value (graph 310), there are jumps in the calculated values in parts, however, this is mostly during normal/expected transitions between sleep stages. As shown, an elevated information entropy level (average) is observed for the patient with moderate sleep apnea (e.g., see, graph 405, patient 2). A high information entropy level (average) is observed for the patient with severe sleep apnea (e.g., see, graph 505, patient 3).

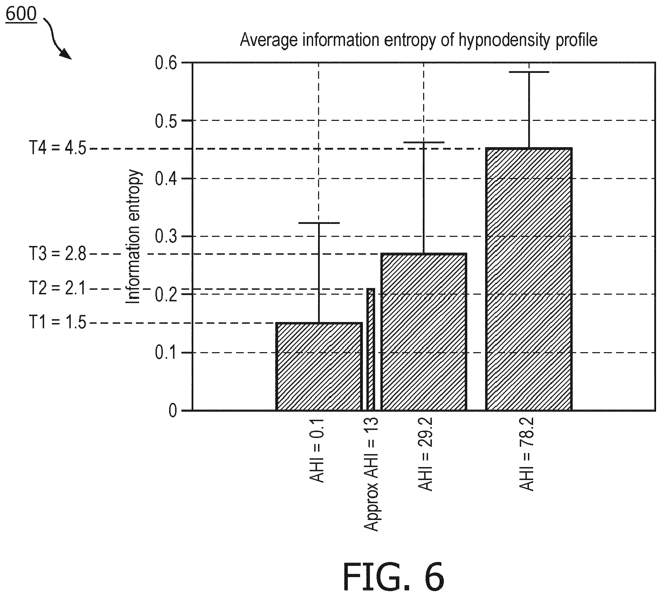

FIG. 6 shows an illustrative graph 600 of information entropy of the hypnodensity profiles (mean $\pm$ standard deviation (STD)) for the three patients (e.g., patients 1, 2, 3) with respective AHI scores in accordance with embodiments of the present system. The average entropy level of the entire sleep session for the three patients from which the graphs 300, 400, and 500 were derived is shown. Clearly, there is a positive relation between the information entropy level and the AHI. This relationship may be mapped to the information entropy such as the average information entropy of the hypnodensity profile. Thus, in accordance with embodiments of the present system, a correlation between the AHI assessment and the average information entropy of the hypnodensity profile may be determined and a model may be formed based upon this correlation.

With regard to the aforesaid mapping of information entropy to the AHI ratings, in accordance with embodiments of the present system, sleep apnea categorization (SAC) may correspond with the following equation:

$$(1) \text{ Normal: SAC} < T1; (2) \text{ Mild sleep apnea: } T1 \leq \text{SAC} < T2; (3) \text{ Moderate sleep apnea:}$$

$$T2 \leq \text{SAC} < T3; \text{ and } (4) \text{ Severe sleep apnea: SAC} \geq T3; \hspace{2cm} \text{(Equation 2)}$$

where thresholds T1, T2, T3, and T4 may correspond with information entropy values which may be set such that the SAC may correspond with (e.g., be mapped to) the known sleep apnea assessments for patients 1 through 3. In accordance with embodiments, the system is not restricted to the four (4) thresholds illustratively shown as less or more severity levels may be defined. In the example provided to simplify the discussion, assuming that patient 1 is normal, then the system may set the value of T1 to 1.5 which is the upper limit of the average information entropy of the hypnodensity profile for patient 1. Similarly, assuming that patient 2 has moderate apnea then the system may set the value of T2 = 2.1 and T3 = 2.8 which are the lower limit and the upper limit, respectively, of the average information entropy of the hypnodensity profile for patient 2. Then, assuming that patient 3 has severe apnea, then the system may set the value of T3 = 2.8 and T4 = 4.5 which are the lower limit and the upper limit, respectively, of the average information entropy of the hypnodensity profile for patient 3. As there are no other patients, T4 does not matter for the sake of this discussion as there are no classifications greater than severe in the current embodiments. For the sake of clarity, and without limitation, the value of T2 is calculated using an approximate AHI for mild apnea. Illustratively, FIG. 6 is shown based on data of only 3 patients, with one interpolated data point for a fourth candidate/patient. In accordance with embodiments of the present system, thresholds for T1, T2, T3, T4 may be based on more data (100's of patients, 1000's of patients, 10,000's of patients, etc.). The process may train itself using training data to set and/or reset the values of T1 through T4 using patients with previously known (e.g., manually assigned) sleep stage assessments. Thereafter, after determining the average information entropy of a hypnodensity profile for a patient, the system may classify the patient using the thresholds set by the current model.

In accordance with embodiments of the present system, the information entropy is just one example of a feature that may be derived from the hypnodensity profile. The features, for example, may be calculated using the entire test period (e.g., sleep session) and/or other time window(s) (e.g., second to third hour of time period, etc.). Further, different statistics of these features may be calculated and used for sleep apnea assessment such as mean, median, std, mad, range, min, max, etc. of measured signals. In addition, while the metrics discussed are calculated for each epoch, it is also possible to look at the dynamics of a hypnodensity profile. For example, in accordance with embodiments of the present system, the number of dominant sleep stage transitions, the average duration of sleep stage cycles, number of awakenings, number of cycles, and other metrics may be derived to access a physical condition, such as sleep apnea.

Referring back to FIG. 2, after completing act 209, the process may continue to act 211 during which a risk assessment may be performed of sleep apnea presence and severity level, if detected. During this act, the hypnodensity-based features and statistics calculated during act 209 may be combined in a model (e.g., as may be selected by the SSI) to provide an estimation of the AHI or another metric representative for the presence/severity of a physical condition, such as sleep apnea. Linear regression or other modelling techniques may be employed for this purpose. The graph 600 of FIG. 6 illustrates an example of a single parameter (e.g., average information entropy) that may be used to estimate the corresponding AHI, however, multiple parameters may be analyzed/combined to improve the accuracy during a training mode and/or to determine whether a condition such as sleep apnea is present for a patient. A thin bar is shown above columns to represent the standard deviation (STD) of the information entropy timeseries. The STD gives an indication of the variability of the entropy within a subject. In FIG. 6, it is plotted to show that despite the variability, there is a clear difference in entropy between the subjects. AHI is a well-established index used by clinicians and is a direct measure of the severity of sleep apnea. AHI linearly increases with the number of apneas/hypopneas during sleep, so it may be helpful for clinicians and patients to get an estimate for AHI. However, in accordance with embodiments of the present system, for example making a categorization between normal, moderate, and severe via thresholds may also be suitably applied. For some of these other indicators, the AHI may be more granular and allow for more easily tracking of changes in severity over time. In addition, a combination of the metrics may be suitably applied. In addition, risk factor information for sleep apnea may be combined in a model with the sensor data to increase the accuracy of a determination.

Accordingly, the sleep apnea prediction model of the present embodiments may also be augmented with other sources of data and sleep apnea risk factors to increase the diagnostic accuracy (e.g., information on snoring, breathing, gasping, body movements, obesity, neck circumference, age, gender, etc.). The presence of certain risk factors may also be utilized to increase the prediction confidence that the patient falls in a certain severity class. For example, the risk factors may be added to a model as additional input parameters (e.g., an additional offset or scaling in a regression model). Some of these parameters are categorical (e.g., gender, yes/no smoking, etc.) which may for instance be coded as 0 and 1 in a model in accordance with the present system. Also, logistic regression and classification methods may be used to perform a risk assessment. Instead of providing a continuous output metric (like AHI), a classification may be performed in this case (e.g., no sleep apnea, mild, moderate, severe). For example, while particular thresholds are illustratively discussed, the actual threshold values may depend on the modeling approach and data being used. Further, instead of providing a continuous output like AHI (e.g., using linear regression modeling), a modeling approach like (multivariable) logistic regression may be used to predict the class/severity.

An advantage of embodiments of the present system is that it is relatively simple, unobtrusive, and may be applied in a home setting. The sleep apnea risk assessment may therefore be performed regularly, e.g., during multiple days during the week. This may provide more insights regarding dynamic characteristics of the presence and severity level of sleep apnea. Data over an extended period of time may also be used to analyze the effects of treatments on the severity of the sleep apnea. For example, in milder cases, lifestyle changes may be sufficient to minimize the obstructive sleep apnea. By tracking the changes in lifestyle, changes in the severity scores may be correlated to determine whether the changes in lifestyle are producing a desired reduction in the severity scores. Accordingly, the respiratory status of the patient which may be stored in the SMI may be updated to reflect the results of the process (e.g., the current sleep apnea test). For example, if severe sleep apnea is detected, the SMI may be updated accordingly.

After completing act 211, the process may continue to act 213 during which results of the sleep apnea test may be rendered on a rendering device of the system. For example, a controller of the system may transmit information corresponding to the results of the process to a rendering device of the system (e.g., the display of the MS), which rendering device may then render this information such as "sleep apnea detected" for the convenience of the viewer. The results may be transmitted to one or more devices of the system such as the CSC, the MS of the patient, and/or the MS of a clinician as may be set in the SSI and may be stored in the SMI that may be updated with the most recent test results. After completing act 213, the process may continue to act 215 during which the updated SMI may be saved. Accordingly, data generated by the process and/or results of the process may be stored in a memory of the system. After completing act 215, the process may continue to act 217 where it may end.

Embodiments of the present system may be integrated into various sleep apnea solutions and sleep monitoring solutions. Next to the home monitoring setting, the solution may also be used to screen for sleep apnea in hospitals and other care settings including sleep study centers. As operations may be automated, a clinician may not be necessary for performing sleep analysis using embodiments of the present system. Further, embodiments of the present system may, depending upon embodiment, employ fewer sensors than employed by conventional systems which enhances user comfort and/or convenience and will reduce costs as compared to the conventional systems. Further variations of the present system would readily occur to a person of ordinary skill in the art and are encompassed by the following claims.

Finally, the above discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art including using features that are described with regard to a given embodiment with other envisioned embodiments without departing from the broader and intended spirit and scope of the present system as set forth in the claims that follow. In addition, any section headings included herein are intended to facilitate a review but are not intended to limit the scope of the present system. In addition, the specification and drawings are to be regarded in an illustrative manner and are not intended to limit the scope of the appended claims.

In interpreting the appended claims, it should be understood that:

a) the word "comprising" does not exclude the presence of other elements or acts than those listed in a given claim;

b) the word "a" or "an" preceding an element does not exclude the presence of a plurality of such elements;

c) any reference signs in the claims do not limit their scope;

d) several "means" may be represented by the same item or hardware or software implemented structure or function;

e) any of the disclosed elements may be comprised of hardware portions (e.g., including discrete and integrated electronic circuitry), software portions (e.g., computer programming), and any combination thereof;

f) hardware portions may be comprised of one or both of analog and digital portions;

g) any of the disclosed devices, features and/or portions thereof may be combined together or separated into further portions unless specifically stated otherwise;

h) no specific sequence of acts or steps is intended to be required unless specifically indicated; and

i) the term "plurality of" an element includes two or more of the claimed element, and does not imply any particular range of number of elements; that is, a plurality of elements may be as few as two elements, and may include an immeasurable number of elements.

**Claims**

1. A sleep monitoring system (100), comprising:

   at least one sensor (114) configured to sense physiological changes of a subject during a test period, the test period having a plurality of epochs, and being configured to form corresponding sensor information for the plurality of the epochs;
   at least one controller (120, 190, 194) configured to:

   receive the sensor information for the plurality of the epochs during the test period from each of the at least one sensor;
   derive a hypnodensity profile for each epoch of the plurality of epochs based upon the received sensor information for the plurality of the epochs;
   calculate at least one hypnodensity feature based upon the hypnodensity profile for each epoch of the plurality of epochs;
   determine a composite feature based on the at least one hypnodensity feature over the plurality of epochs;
   compare the composite feature to a at least one predetermined indicator related to sleep apnea; and
   determine a presence and/or severity of apnea based upon the comparison.

2. The sleep monitoring system of claim 1, wherein at least one of the at least one sensor (114) is configured to measure sleep.

3. The sleep monitoring system of claim 1, wherein the at least one controller is further configured to monitor the at least one sensor in accordance with a test type.

4. The sleep monitoring system of claim 1, further comprising a mobile station (MS) having a display, wherein the at least one controller is further configured to transmit the determined presence and/or severity of apnea to the MS and to render the determined presence and/or severity of apnea on the display.

5. The sleep monitoring system of claim 1, wherein the at least one controller is configured to calculate the at least one hypnodensity feature as information entropy, and is configured to determine an average of the information entropy (DA) over the plurality of epochs as the composite feature.

6. The sleep monitoring system of claim 5, wherein the at least one predetermined indicator is at least one predetermined threshold for the presence and/or the severity of apnea.

7. The sleep monitoring system of claim 6, wherein the hypnodensity profile is derived using a model of sleep classes, wherein the model is determined from training data from a population in which at least some of the population are determined to exhibit at least some degree of sleep apnea, and wherein the at least one predetermined threshold is determined from the training data.

8. The sleep monitoring system of claim 7, wherein the at least one predetermined threshold is a first threshold value (T1), a second threshold value (T2), and a third threshold value (T3), wherein the controller is further configured to compare the DA to the T1, T2, T3 and T4 wherein: when it is determined that the DA is less than T1, then no apnea is determined present; when it is determined that the DA is equal or greater than T1 and less than T2, then mild apnea is determined present; when it is determined that the DA is equal or greater than T2 and less than T3, then moderate apnea is determined present; and when it is determined that the DA is equal or greater than T3, severe apnea is determined present.

9. A sleep monitoring system, comprising:

at least one sensor (114) configured to sense physiological changes of a subject during a test period having a plurality of epochs and form corresponding sensor information for the plurality of the epochs;
at least one controller (120, 190, 194) configured to:

receive the sensor information for the plurality of the epochs during the test period from each of the plurality of sensors;
derive a hypnodensity profile for each epoch of the plurality of epochs based upon the received sensor information for the plurality of the epochs;
calculate at least one hypnodensity feature based upon the hypnodensity profile for each epoch of the plurality of epochs;
form a sleep apnea prediction model based upon the calculated at least one hypnodensity feature;
determine a presence and/or severity of apnea based upon the sleep apnea prediction model; and
render the results of the determined presence and/or severity of apnea on a rendering device.

10. The sleep monitoring system of claim 9, wherein at least one of the at least one sensor (114) is configured to measure sleep.

11. The sleep monitoring system of claim 9, further comprising a mobile station (MS) (104) having a display (192), wherein the at least one controller is further configured to transmit the determined presence and/or severity of apnea to the MS and to render the determined presence and/or severity of apnea on the display.

12. The sleep monitoring system of claim 9, wherein the at least one controller is further configured to determine a composite feature based on the at least one hypnodensity feature over the plurality of epochs; and form the sleep apnea prediction based upon the calculated at least one hypnodensity feature and the composite feature.

13. A method of monitoring sleep, the method comprising acts of:

sensing physiological changes of a subject during a test period, the test period having a plurality of epochs and forming corresponding sensor information for the plurality of the epochs;
receiving the sensor information for the plurality of the epochs during the test period from each of the at least one sensor;
deriving a hypnodensity profile for each epoch of the plurality of epochs based upon the received sensor information for the plurality of the epochs;
calculating at least one hypnodensity feature based upon the hypnodensity profile for each epoch of the plurality of epochs;
determining a composite feature based on the at least one hypnodensity feature over the plurality of epochs;
comparing the composite feature to a at least one predetermined indicator related to sleep apnea; and
determining a presence and/or severity of apnea based upon the comparison.

14. The method of monitoring sleep of claim 13, further comprising acts of:

calculating the at least one hypnodensity feature as information entropy; and
determining an average of the information entropy (DA) over the plurality of epochs as the composite feature.

15. The method of monitoring sleep of claim 13, further comprising an act of determining at least one threshold, wherein the hypnodensity profile is derived using a model of sleep classes, wherein the model is determined from training data from a population in which at least some of the population are determined to exhibit at least some degree of sleep apnea, and wherein the at least one threshold is determined from the training data.

FIG. 1

FIG. 2

Patient 1 with AHI = 0.1

FIG. 3

FIG. 4

FIG. 5

FIG. 6

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

**EP 23 15 4473**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/405222 A1 (DE GROOT KOEN THEO JOHAN [NL] ET AL) 31 December 2020 (2020-12-31) | 1-4,13 | INV. A61B5/024 A61B5/318 |
| Y | * paragraph [0098] - paragraph [0108] * * paragraph [0114] - paragraph [0122] * | 5-7,14, 15 | A61B5/369 A61B5/389 |
| A | * paragraph [0140] * * figures 2, 4 * ----- | 8 | A61B5/398 A61B5/00 |
| Y | US 2017/055898 A1 (BANDYOPADHYAY AMRIT [US] ET AL) 2 March 2017 (2017-03-02) * paragraph [0106] - paragraph [0121] * ----- | 5-7,14, 15 | |
| A | US 2008/243017 A1 (MOUSSAVI ZAHRA [CA] ET AL) 2 October 2008 (2008-10-02) * paragraph [0097] - paragraph [0103] * ----- | 8 | |

TECHNICAL FIELDS
SEARCHED      (IPC)

**A61B**

### INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 16 October 2023 | Gooding Arango, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.......................................................................
& : member of the same patent family, corresponding
    document

EPO FORM 1503 03.82 (P04E07)

Europäisches
Patentamt

European
Patent Office

Office européen
des brevets

**INCOMPLETE SEARCH
SHEET C**

Application Number

**EP 23 15 4473**

```
Claim(s) completely searchable:
     1-8, 13-15

Claim(s) not searched:
     9-12

Reason for the limitation of the search:

The search was carried out according to the reply of the applicant to the
invitation pursuant to Rule 62a(1) EPC, namely the apparatus of
independent claim 1, its dependent claims and the corresponding method
claims.
```

## ANNEX TO THE EUROPEAN SEARCH REPORT
## ON EUROPEAN PATENT APPLICATION NO.

EP 23 15 4473

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2023

| Patent document cited in search report | | Publication date | Patent family member(s) | | Publication date |
|---|---|---|---|---|---|
| US 2020405222 | A1 | 31-12-2020 | CN | 111867450 A | 30-10-2020 |
| | | | EP | 3536225 A1 | 11-09-2019 |
| | | | EP | 3761852 A1 | 13-01-2021 |
| | | | JP | 2021517008 A | 15-07-2021 |
| | | | US | 2020405222 A1 | 31-12-2020 |
| | | | WO | 2019170734 A1 | 12-09-2019 |
| US 2017055898 | A1 | 02-03-2017 | US | 2017055898 A1 | 02-03-2017 |
| | | | WO | 2017040331 A1 | 09-03-2017 |
| US 2008243017 | A1 | 02-10-2008 | US | 2008243014 A1 | 02-10-2008 |
| | | | US | 2008243017 A1 | 02-10-2008 |
| | | | WO | 2008116318 A1 | 02-10-2008 |

EPO FORM P0459

For more details about this annex : see Official Journal of the European Patent Office, No. 12/82